# EUROPEAN PATENT APPLICATION

(11) **EP 2 476 409 A1**
(43) Date of publication of application: **18.07.2012**
(21) Application number: 11151024.4
(22) Date of filing: 14.01.2011
(51) Int. Cl.: A61K 9/00, A61K 31/19, A61K 31/415, A61K 31/4196, A61K 31/565

(54) **Implant comprising a core and a tube encasing the core**

(71) Applicant: Université Catholique de Louvain, 1348 Louvain-La-Neuve (BE); Université de Liège, 4031 Angleur (BE)
(72) Inventor: Donnez, Jacques, 1150, Bruxelles (BE); Van Langendonkt, Anne, 1070, Bruxelles (BE); Defrere, Sylvie, 7822, Isières (BE); Foidart, Jean-Michel, 4870, Forest-Trooz (BE); Jerome, Christine, 4102, Ougrée (BE); Evrard, Brigitte, 4053, Embourg (BE); Riva, Raphael, 4400, Flémalle (BE); Krier, Fabrice, 4040, Herstal (BE); Mestdagt, Mélanie, 4122, Plainevaux (BE)
(74) Representative: Bounaga, Sakina

(57) **Abstract**

The present invention relates to an implant comprising:
- a core material comprising polydimethylsiloxane or at least one hydrogel polymer;
- a tube encasing said core material comprising an ethylene vinyl acetate polymer or at least one hydrogel polymer;
- a sealant for closure of the open ends of said tube comprising polydimethylsiloxane or a mono-, di-, or triacetoxy derivative thereof, or at least one hydrogel polymer; and
- at least one active ingredient;
with the proviso that when the sealant is said at least one hydrogel polymer, the core material comprises polydimethylsiloxane.

Furthermore, the invention relates to an implant for use as a medicament. In particular, the invention relates to an implant for use in the treatment of endometriosis.

## Description

### FIELD OF THE INVENTION

The present invention relates to an implant of polymeric material. Furthermore, the invention relates to an implant for use as a medicament. In particular, the invention relates to an implant for use in the treatment of endometriosis.

### BACKGROUND OF THE INVENTION

Endometriosis is a gynecological disorder characterized by the presence of endometrial tissue outside the endometrial cavity, most commonly in the abdominal cavity. The ectopic endometrial tissue remains hormone responsive, such as cyclical bleeding and estrogen-dependent growth. The ectopic growth triggers abdominal pain leading to a loss in quality of life, and immune system activation. Frequently, endometriosis leads to infertility in affected women. Because endometriosis is often confined to the peritoneal cavity, localized drug delivery into this cavity is of great interest for the treatment of endometriosis and in general for the treatment of pathologies confined to the peritoneal cavity.

Implants of polymeric material as drug delivery systems are known for some time. Implantable delivery systems of polymeric material are known for instance for the delivery of contraceptive agents. However, prior art implants do not sufficiently control drug release. Various devices have been proposed for solving this problem. However, none have been entirely satisfactory. For example, US Patent N° 6,117,441 discloses an implantable system for use as a male contraception and as a treatment of benign prostate hypertrophy and other conditions.

Accordingly, a need exists for improved polymeric implants. In particular, there remains a need for an implant with controlled drug release for use in the treatment of endometriosis. It is an object of the invention to provide an implant with controlled drug release.

### SUMMARY OF THE INVENTION

In a first aspect, the present invention provides implants for extended release of an active ingredient, comprising a core material comprising polydimethylsiloxane (PDMS) or at least one hydrogel polymer; a tube encasing said core material comprising an ethylene vinyl acetate polymer or at least one hydrogel polymer; a sealant for closure of the open ends of said tube comprising polydimethylsiloxane or a mono-, di-, or triacetoxy derivative thereof, or at least one hydrogel polymer; and at least one active ingredient; with the proviso that when the sealant is said at least one hydrogel polymer, the core material comprises polydimethylsiloxane.

The present inventors have found that an implant according to the invention has the advantage of overcoming one or more of the above-mentioned problems of the prior art. The present implants of the invention have the advantage of allowing controlled liberation of active ingredients over extended periods of time and hence increase patient compliance during long-term treatment. Controlled drug release allows the sustained delivery of the drug in a predetermined amount and this during a defined period of time. Furthermore, the present implants protect their enclosed active ingredient from the physical environment, thereby improving active ingredient stability in vivo. Furthermore, in an embodiment, the implant of the present invention can be easily localized in the body, due to the presence of a radiopaque material and/or an inert metal coating. This is advantageous at the time of implantation and after the treatment in order to facilitate the removal of the implant.

In a second aspect, the present invention relates to an implant for use as a medicament. In particular, the invention relates to an implant for use in the treatment of endometriosis. The use of implants of the present invention is advantageous because these implants allow efficient treatment while avoiding side effects, due to the sustained and localized delivery of a therapeutically effective amount of the enclosed drug. Furthermore, the use of implants of the present invention is advantageous because these implants allow treatment during longer periods, due to the controlled release of the active ingredient. The invention therefore also relates to an implant according to the invention for use as a medicament, wherein said implant is administered once per 180 days, or less frequently, preferably once per year, or less frequently. A further advantage of the use of the present implants is that these implants allow simultaneous delivery of several active ingredients of different therapeutic classes.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### BRIEF DESCRIPTION OF THE FIGURES

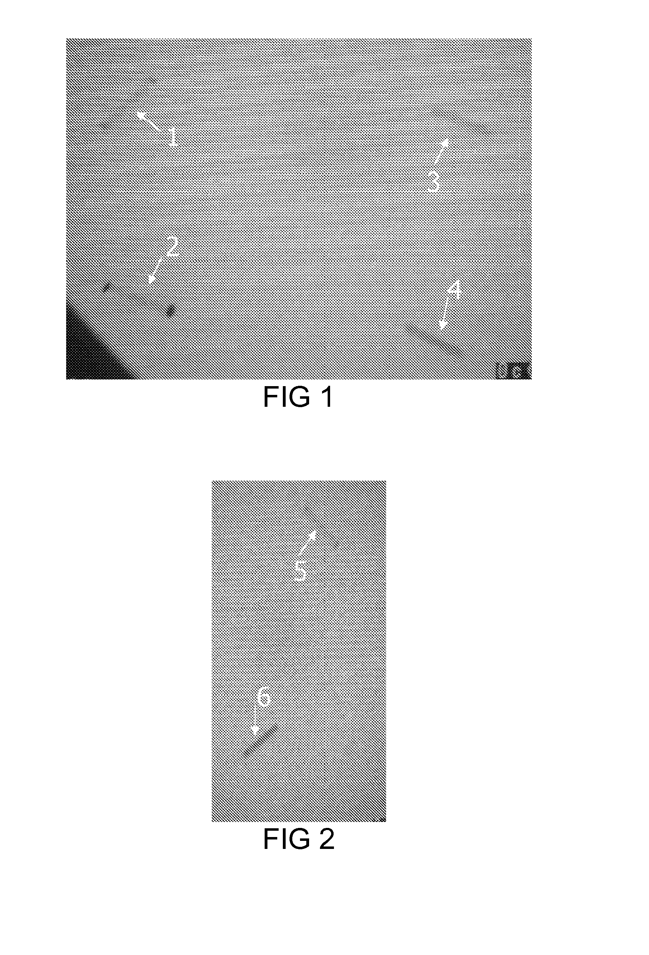

Figures 1 and 2 represent X-ray images of implants comprising barium sulfate.

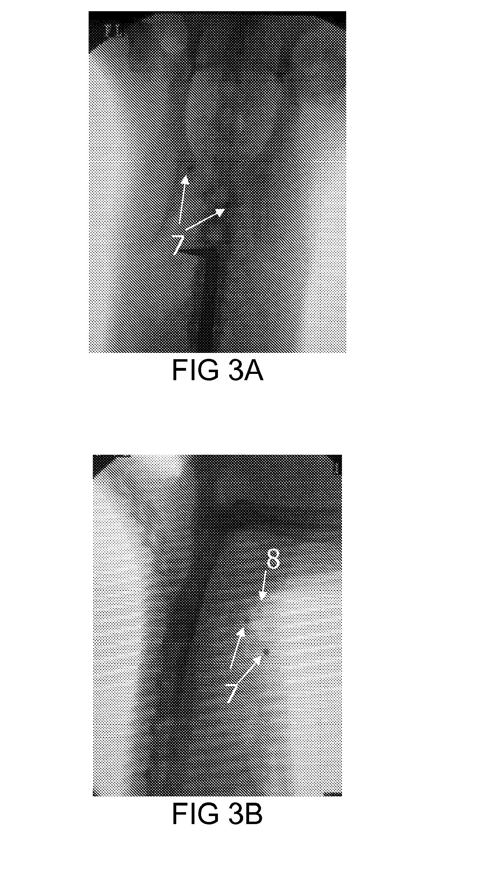

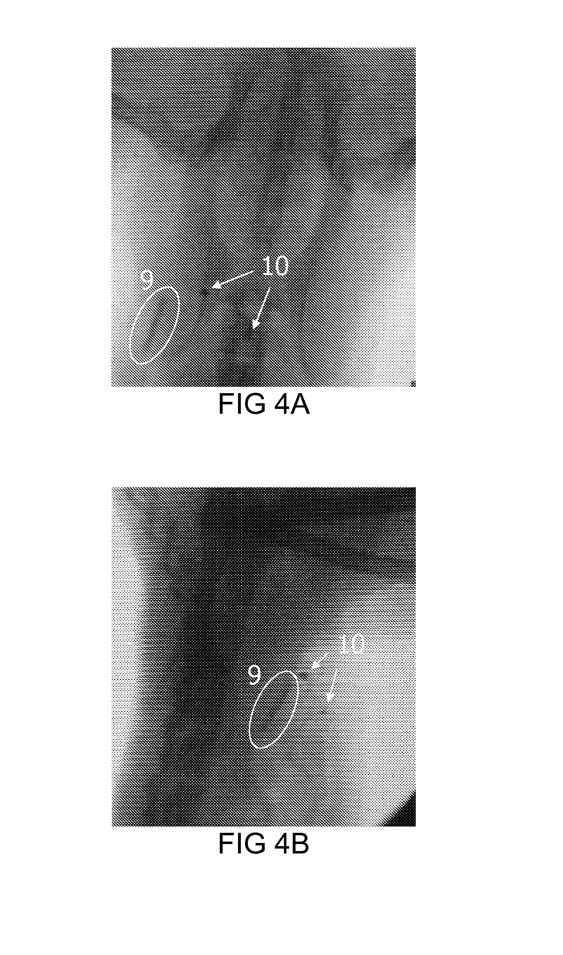

Figures 3, 4 and 5 represent X-ray images of in vivo implants comprising barium sulfate. Figure 3A represents the front view of the animal on day 0. Figure 3B represents the side view of the animal on day 0. Figure 4A represents the front view of the animal on day 0.

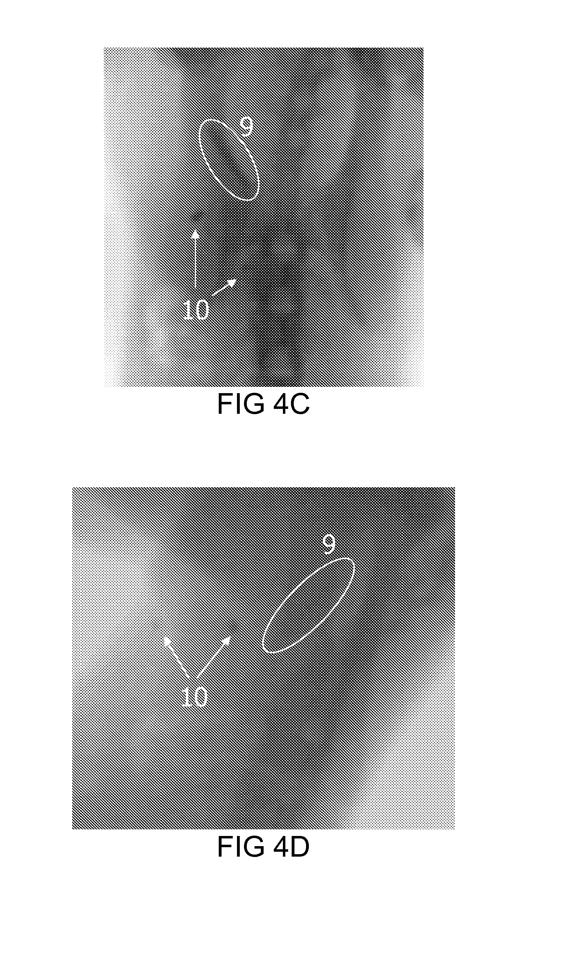

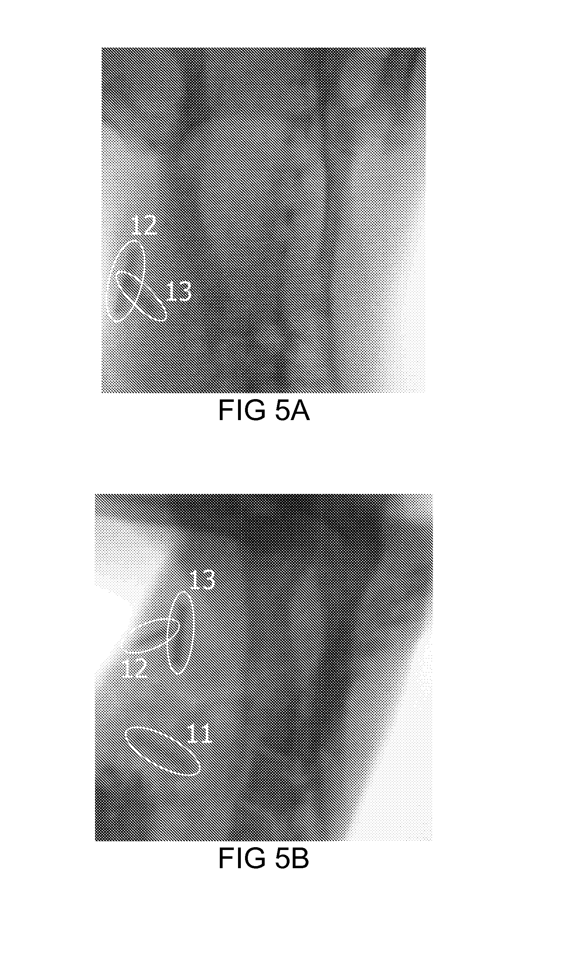

Figure 4B represent the side view of the animal on day 0. Figure 4C represents the front view of the animal after 2 months. Figure 4D represents the side view of the animal after 2 months. Figure 5A represents the front view of the animal on day 0. Figure 5B represents the side view of the animal on day 0.

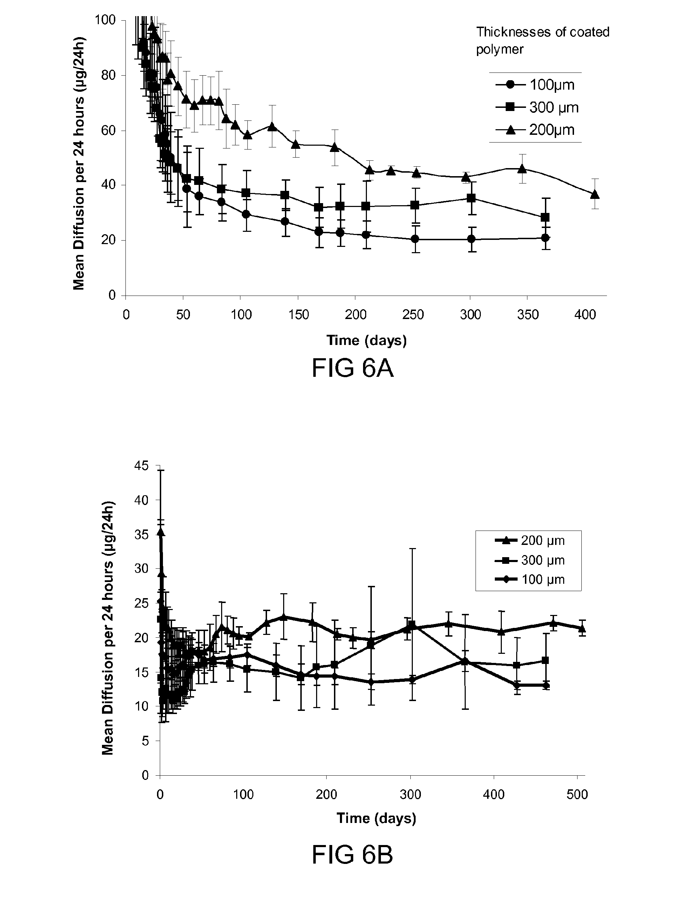

Figure 6A represents a graph illustrating the mean release of anastrozole per 24h in function of the time for an implant without a sealant. Figure 6B represents a graph illustrating the mean release of anastrozole per 24h in function of the time for an implant with MED-2000 adhesive silicone as a sealant.

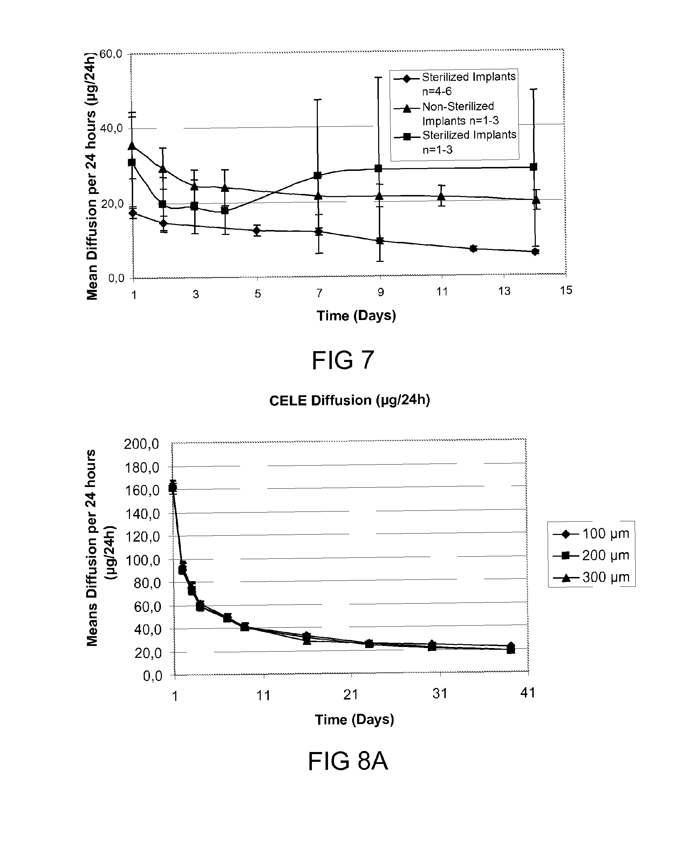

Figure 7 represents a graph illustrating the mean release of anastrozole per 24h in function of the time for sterilized and non-sterilized implants.

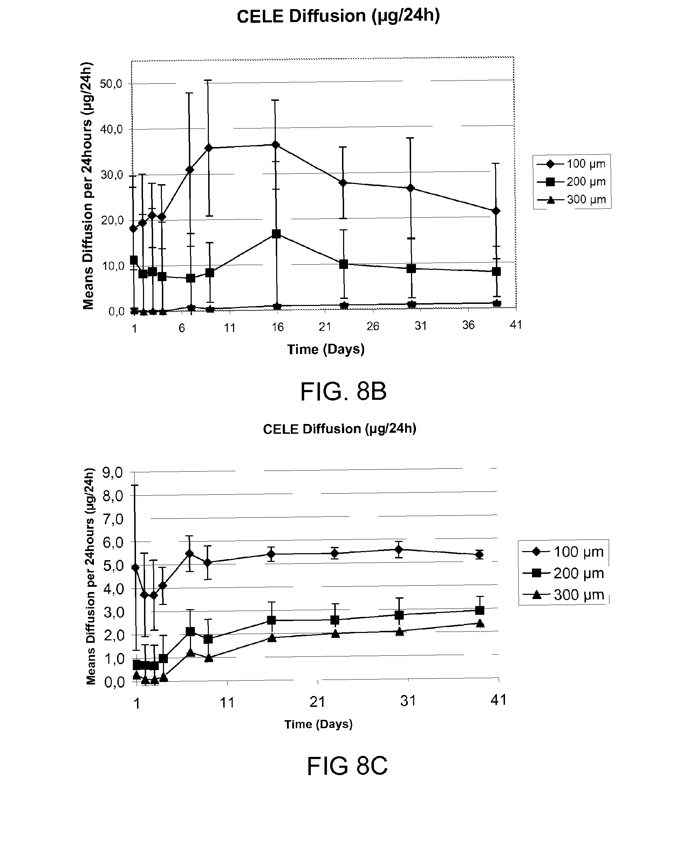

Figure 8A: represents a graph illustrating the mean release of celecoxib per 24h in function of the time for implants without a sealant and with an EVA (10% of VA) membrane of different thicknesses. Figure 8B: represents a graph illustrating the mean release of celecoxib per 24h in function of the time for implants with an EVA sealant and with an EVA (10% of VA) membrane of different thicknesses. Figure 8C: represents a graph illustrating the mean release of celecoxib per 24h in function of the time for implants with a PDMS sealant and with an EVA (10% of VA) membrane of different thicknesses.

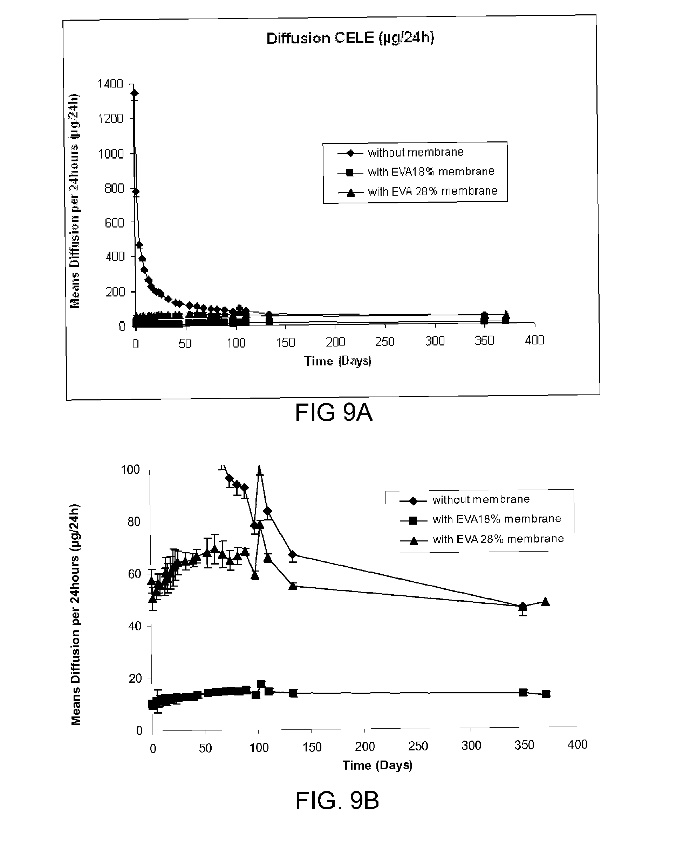

Figure 9A represents a graph illustrating the mean release of celecoxib per 24h in function of the time for implants without a tube and with an EVA tube comprising 18% or 28% by weight of vinyl acetate. Figure 9B is a close-up view of figure 9A

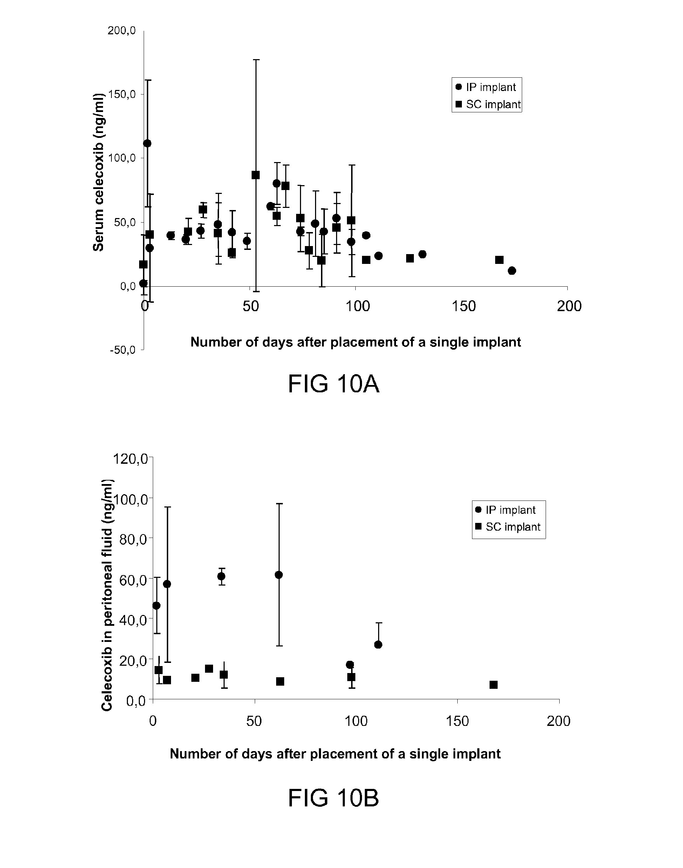

Figure 10A represents a graph illustrating the concentration of celecoxib in serum of rats in function of the number of days after implantation. Figure 10B represents a graph illustrating the concentration of celecoxib in peritoneal liquid of rats in function of the number of days after implantation.

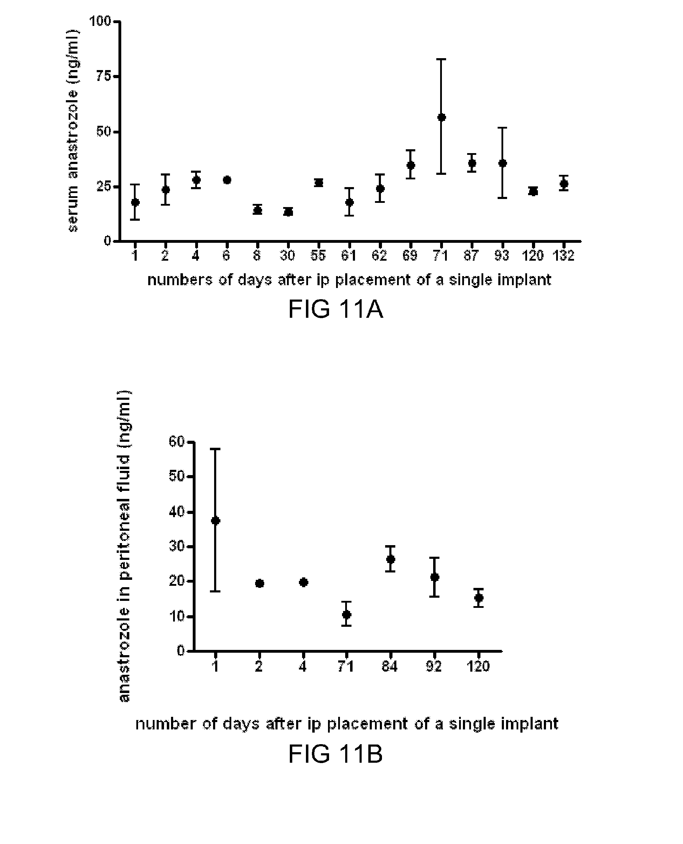

Figure 11A represents a graph illustrating the concentration of anastrozole in serum of rats in function of the number of days after intraperitoneal implantation. Figure 11B represents a graph illustrating the concentration of anastrozole in peritoneal fluid of rats in function of the number of days after intraperitoneal implantation.

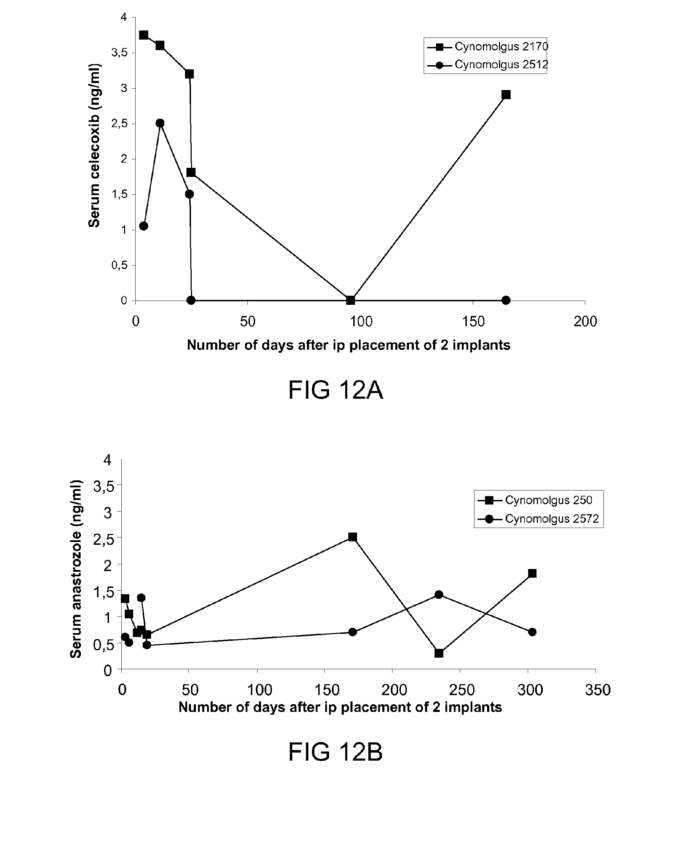

Figure 12A represents a graph illustrating the concentration of celecoxib in serum of cynomolgus monkeys in function of the number of days after implantation. Figure 12B represents a graph illustrating the concentration of anastrozole in serum of cynomolgus monkeys in function of the number of days after implantation.

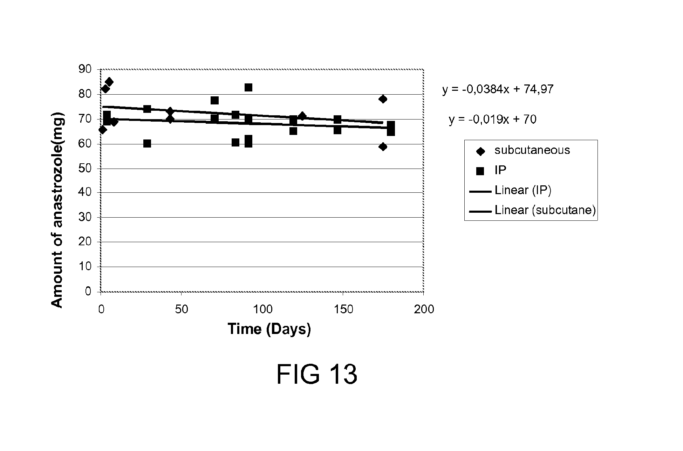

Figure 13 represents a graph illustrating the concentration of anastrozole in the implants placed subcutaneously or intraperitoneally in function of the time.

### DETAILED DESCRIPTION OF THE INVENTION

In the following passages, different aspects of the invention are described in more detail. Each aspect so described may be combined with any other aspect or aspects unless clearly indicated to the contrary. In particular, any feature indicated as being preferred or advantageous may be combined with any other feature or features indicated as being preferred or advantageous.

In the context of the present invention, the terms used are to be construed in accordance with the following definitions, unless a context dictates otherwise.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

In a first aspect, the present invention provides to an implant for delivering at least one active ingredient, said implant comprising: a core material comprising polydimethylsiloxane (PDMS) or at least one hydrogel polymer; a tube encasing said core material comprising an ethylene vinyl acetate polymer or at least one hydrogel polymer; and a sealant for closure of the open ends of said tube comprising PDMS or a mono-, di-, or triacetoxy derivative thereof, or at least one hydrogel polymer; and at least one active ingredient; with the proviso that when the sealant is said at least one hydrogel polymer, the core material comprises PDMS.

According to an embodiment of the invention, the core material of the implants of the invention is composed of polydimethylsiloxane (PDMS). Preferably, medical grade PDMS is used. Suitable non-limiting example of medical grade PDMS which can be used as a core material is for instance PDMS with the designations MED-4211, MED-4244, MED-4286, MED-4420, MED-6210, MED-6215, MED-6219, MED-6233, MED-6385, MED-6820 and MED-6380 (Nusil technology, Carpinteria, CA, USA).

In some embodiments, the core material of the present implant comprises from about 25 % to about 60% by weight of PDMS. For example, the core material comprises from about 30% to about 60% by weight of PDMS, for example from about 35% to about 60% by weight of PDMS, for example from about 40% to about 60% by weight of PDMS, for example from about 45% to about 60% by weight of PDMS, for example from about 50% to about 60% by weight of PDMS, for example from about 55% to about 60% by weight of PDMS.

In another embodiment, the core material of the present implant comprises at least one hydrogel polymer. Various hydrogel polymers can be used, such as those obtained by homopolymerization or copolymerization of 2-hydroxyethyl methacrylate (HEMA), hydroxypropyl methacrylate (HPMA) or ethylene glycol dimethacrylate (EGDMA). In some embodiments, said hydrogel polymer comprises from about 99% to about 99.9% by weight of HEMA and from about 0.1% to about 1% by weight of EGDMA. In another embodiment, said hydrogel polymer comprises from about 95% to about 50% by weight of HEMA, from about 5% to about 50% by weight of HPMA and from about 0.1% to about 1% by weight of EGDMA. In a preferred embodiment, said hydrogel polymer comprises about 99.9% by weight of HEMA and about 0.1% by weight of EGDMA.

In some embodiments, the invention provides an implant, wherein the core material comprises from about 25% to about 60% by weight of at least one hydrogel polymer. For example, the core material comprises from about 30% to about 60% by weight of hydrogel polymer, for example from about 35% to about 60% by weight of hydrogel polymer, for example from about 40% to about 60% by weight of hydrogel polymer, for example from about 45% to about 60% by weight of hydrogel polymer, for example from about 50% to about 60% by weight of hydrogel polymer, for example from about 55% to about 60% by weight of hydrogel polymer.

In an embodiment, the tubes encasing the core material of the implants of the invention comprise an ethylene vinyl acetate (EVA) polymer. In an embodiment, the EVA polymer has a vinyl acetate content of less than 45% by weight. Preferably, the EVA polymer has a vinyl acetate content of between 5 and 40% by weight. For example, the EVA polymer has a vinyl acetate content of between 7% and 40% by weight, for example between 7% and 35% by weight, preferably between 7% and 30% by weight, preferably between 7% and 20% by weight, preferably between 7% and 10% by weight. More preferably, the EVA polymer has a vinyl acetate content of 5, 6, 7, 7.5, 10, 15, 18, 20 25, 28, or 30% by weight. In a further embodiment, the ethylene vinyl acetate polymer has a melt index of less than 10g/10 min, and preferably less than or equal to 8g/10 min. Suitable EVA polymers which can be used as a membrane are for instance Evatane® (Arkema) with the designations 501/502 (melt index 2, vinyl acetate content 7.5%), 554/555 (4, 12.5%), 540 (10, 18%), 571 (8, 15%), 1080 VN 5 and 1040 VN 4 and Elvax® (Dupont) with the designations 450, 460, 470, 550, 560, 650, 660, 670, 750, 760, 770 and in particular 3120, 3124, 3128, 3129, 3130, 3150, 3165, 3170, 3174, 3180, 3182, 3185 and 3190.

In another embodiment, the tubes encasing the core material of the implants of the invention comprise at least one hydrogel polymer. Various hydrogel polymers can be used, such as those obtained by homopolymerization or copolymerization of 2-hydroxyethyl methacrylate (HEMA), hydroxypropyl methacrylate (HPMA) or ethylene glycol dimethacrylate (EGDMA). In some embodiments, said hydrogel polymer comprises from about 99% to about 99.9% by weight of HEMA and from about 0.1% to about 1% by weight of EGDMA. In another embodiment, said hydrogel polymer comprises from about 95% to about 50% by weight of HEMA, from about 5% to about 50% by weight of HPMA and from about 0.1% to about 1% by weight of EGDMA. In a preferred embodiment said hydrogel polymer comprises about 99.9% by weight of HEMA and about 0.1% by weight of EGDMA.

In some embodiments, the tubes encasing the core material of the implants and comprising an EVA polymer have a thickness ranging from about 100 µm to about 300 µm. For example, the tubes comprising an EVA polymer have a thickness ranging from about 150 µm to 300 µm, for example ranging from about 200 µm to 300 µm, for example ranging from about 250 µm to 300 µm. For example, the tubes comprising an EVA polymer have a thickness of about 100, 110, 120, 130, 140, 150, 160, 170, 180, 190, 200, 210, 220, 230, 240, 250, 260, 270, 280, 290, or 300 µm, or a value in the range between any two of the aforementioned values. Preferably, the tubes comprising an EVA polymer have a thickness of about 200 µm.

In some embodiments, the tubes encasing the core material of the implants and comprising at least one hydrogel polymer have a thickness ranging from about 100 µm to about 600 µm. For example, the tubes comprising at least one hydrogel polymer have a thickness ranging from about 200 µm to about 600 µm, for example ranging from about 300 µm to about 600 µm, for example ranging from about 400 µm to about 600 µm, for example ranging from about 500 µm to about 600 µm. For example, the tubes comprising at least one hydrogel polymer have a thickness of about 100, 150, 200, 250, 300, 350, 400, 450, 500, 550, or 600 µm, or a value in the range between any two of the aforementioned values. Preferably, the tubes comprising at least one hydrogel polymer have a thickness of about 500 µm.

According to a preferred embodiment, the sealant for closure of the open ends of the tubes comprises PDMS or a mono-, di-, or triacetoxy derivative thereof. Preferably, medical grade PDMS or a mono-, di-, or triacetoxy derivative thereof is used. For example, MED-2000 adhesive silicone of Nusil technology (Carpinteria, CA, USA) is used to seal the open ends of the implant. The sealant is chosen in order to control drug release of the implant.

According to an embodiment, the sealant for closure of the open ends of the tubes comprises at least one hydrogel polymer, with the proviso that when the sealant is said at least one hydrogel polymer, the core material comprises PDMS. Various hydrogel polymers can be used as a sealant, such as those obtained by homopolymerization or copolymerization of 2-hydroxyethyl methacrylate (HEMA), hydroxypropyl methacrylate (HPMA) or ethylene glycol dimethacrylate (EGDMA). In an embodiment, said hydrogel polymer comprises from about 99% to about 99.9% by weight of HEMA and from about 0.1% to about 1% by weight of EGDMA. In another embodiment, said hydrogel polymer comprises from about 95% to about 50% by weight of HEMA, from about 5% to about 50% by weight of HPMA and from about 0.1% to about 1% by weight of EGDMA. In a preferred embodiment said hydrogel polymer comprises about 99.9% by weight of HEMA and about 0.1 % by weight of EGDMA.

The present inventors have found that implants according to the present invention allow controlled liberation of the enclosed drug. The implants according to the present invention are designed in order to allow a controlled release of at least one active ingredient over the functional useful life of the implant. This should preferably be at least 180 days and more preferably one year or longer. Implants capable of delivering at least one active ingredient evenly over 180 days and longer are particularly preferred and implants capable of delivering at least one active ingredient evenly over one year or longer are even more particularly preferred.

In an embodiment, the implants of the present invention comprise a PDMS core provided in an EVA tube and a sealant for closure of the open ends of said tube comprising PDMS or a mono-, di-, or triacetoxy derivative thereof. In another embodiment, the implants of the present invention comprise a PDMS core provided in a hydrogel polymer tube and a sealant for closure of the open ends of said tube comprising PDMS or a mono-, di-, or triacetoxy derivative thereof. In another embodiment, the implants of the present invention comprise a hydrogel polymer core provided in a hydrogel polymer tube and a sealant for closure of the open ends of said tube comprising PDMS or a mono-, di-, or triacetoxy derivative thereof. In yet another embodiment, the implants of the present invention comprise a PDMS core provided in a hydrogel polymer tube and a sealant for closure of the open ends of said tube comprising at least one hydrogel polymer.

In an embodiment, the implants are preferably of essentially cylindrical shape with a maximal external diameter of about 4 mm and a length of less than about 5 cm. Preferably, the implants are of cylindrical shape with an external diameter between 2 and 4 mm and a length between 1 and 4 cm. For example, the implants are of cylindrical shape with an external diameter of 2, 2.5, 3, 3.5 or 4 mm, or a value in the range between any two of the aforementioned values, and a length of 1, 1.5, 2, 2.5, 3, 3.5 or 4 cm, or a value in the range between any two of the aforementioned values. More preferable, the implants are of cylindrical shape with an external diameter of about 3 mm and a length of about 2 cm. The diameter of the core material of the implant is obviously sufficient to fit within the tube encasing the core material. Of course, depending upon the circumstances, it may be necessary or desirable to increase the length or diameter of the implant or to change it from a cylindrical configuration to a different geometry. In this regard, other geometric shapes, including, for example, rings, loops, and discs, are contemplated for the present invention. However, as it is necessary to produce the implant in such a way as not to cause an impediment or to cause discomfort to the user, it is preferable to keep it as small and unobtrusive as possible.

In an embodiment, said implant comprises an inert metal coating and/or at least one radiopaque material. In an embodiment, the implant comprises said radiopaque material in the core material or in the sealant of the implant. In another embodiment, the implant comprises said radiopaque material in the core material and in the sealant of the implant.

In an embodiment, said implant comprises at least 0.01% by weight of an inert metal coating and/or at least one radiopaque material.

In an embodiment, said radiopaque material is provided in the core material. In this embodiment the core material can comprise from about 0.01 % to about 60% by weight of radiopaque material, for example from about 0.1 % to about 55% by weight of radiopaque material, for example from about 1% to about 50% by weight, for example from about 1% to about 40% by weight, for example from about 1% to about 30% by weight, for example from about 1% to about 20% by weight of radiopaque material. For example, the core material can comprise about 0.01%, 0.1%, 1%, 5%, 10%, 15%, 20%, 25%, 30%, 35%, 40%, 45%, 50%, 55% or 60% by weight of radiopaque material. In an embodiment, said radiopaque material is provided in the sealant. In this embodiment the sealant can comprise from about 0.01% to about 40% by weight of radiopaque material, for example from about 0.1% to about 35% by weight, for example from about 1% to about 30% by weight, for example from about 1% to about 25% by weight, for example from about 1% to about 20% by weight of radiopaque material. For example, the sealant material can comprise about 0.01%, 0.1%, 1%, 5%, 10%, 15%, 20%, 25%, 30%, 35% or 40% by weight of radiopaque material.

In an embodiment, the radiopaque material may be selected from the group comprising barium, gold, platinum, tantalum, bismuth and iodine or salts thereof, or a radiopaque polymer. The radiopaque material can be incorporated into the implant in several ways. Biocompatible non-immunogenic metals such as gold and platinum may be incorporated as a very fine dispersion with particle sizes less than a few micrometers. Other heavy atoms may be incorporated in the form of inorganic salts, such as barium sulfate. In an embodiment, the radiopaque material is a radiopaque polymer. The radiopaque polymer may also be incorporated in the implant by using radiopaque (meth)acrylic monomers during the preparation of the implant (Saralidze et al., Biomacromolecules 4(3): 793-8, 2003). In an embodiment, said radiopaque (meth)acrylic monomer is 2-[2',3',5'-triiodobenzoyl]oxoethyl methacrylate. This methacrylate is intrinsically radiopaque and capable of absorbing X-radiation.

The incorporation of a radiopaque material in the implant allows localization of the implant in the body. This localization is important to follow the implant during implantation and to allow easy removal of the implant after treatment. The implants of the present invention comprising a radiopaque material can be detected using X-ray techniques. X-ray techniques are performed as known by the skilled man in the art.

In a preferred embodiment, said radiopaque material is barium sulfate. In an embodiment said barium sulfate is provided in the sealant. In this embodiment the sealant can comprise from about 0.01% to about 40% by weight of barium sulfate, for example from about 0.1% to about 35% by weight, for example from about 1% to about 30% by weight, for example from about 1% to about 25% by weight, for example from about 1% to about 20% by weight of barium sulfate. For example, the sealant can comprise about 0.01%, 0.1%, 1%, 5%, 10%, 15%, 20%, 25%, 30%, 35% or 40% by weight of barium sulfate. In an embodiment, said implant comprises at least 0.01% by weight of an inert metal coating and/or at least one radiopaque material. In another embodiment, said barium sulfate is provided in the core material. In this embodiment the core material can comprise from about 0.01 to about 60% by weight of barium sulfate, for example from about 0.1% to about 55% by weight, for example from about 1% to about 50% by weight, for example from about 1% to about 40% by weight, for example from about 1% to about 30% by weight, for example from about 1% to about 20% by weight of barium sulfate. For example, the core material can comprise about 0.01%, 0.1%, 1%, 5%, 10%, 15%, 20%, 25%, 30%, 35%, 40%, 45%, 50%, 55% or 60% by weight of barium sulfate. In yet another embodiment, said barium sulfate is provided in the core material and in the sealant.

In another embodiment, the implant comprises an inert metal coating. The inert metal may be selected from the group comprising silver, gold, titanium, tungsten, barium, bismuth, platinum and palladium. Preferred metals are those known to be compatible with the human body, such as silver, gold, titanium and platinum. The inert metal can be coated on the implant as a fine layer. The thickness of the inert metal layer coated on the implant may be between 0.1 nm and 500 nm. Preferably, the thickness of the inert metal layer coated on the implant may be between 1 nm and 50 nm. For example, the thickness of the inert metal layer coated on the implant may be 1, 5, 10, 15, 20, 25, 30, 35, 40, 45, or 50 nm, or a value in the range between any two of the aforementioned values. The implant comprising an inert metal according to the present invention can be detected using ultrasound techniques. The addition of an inert metal coating on the implant allows localization of the implant in the body. This localization is important to follow the implant during implantation and to allow easy removal of the implant after treatment. Ultrasound techniques are performed as known by the skilled man in the art.

According to the invention, the implant comprises at least one active ingredient selected from an anti-inflammatory agent, a steroid, an aromatase inhibitor or a gonadotropin-releasing hormone agonist. Preferably, the implant comprises at least one active ingredient selected from an anti-inflammatory agent, a steroid, or a gonadotropin-releasing hormone agonist.

In an embodiment, the implant comprises from about 40% to about 75% by weight of at least one active ingredient as defined above. For example, said implant comprises from about 40% to about 70% by weight of at least one active ingredient, for example from about 40% to about 65% by weight, for example from about 40% to about 60% by weight of at least one active ingredient, for example from about 40% to about 55% by weight, for example from about 40% to about 50% by weight of at least one active ingredient. For example, the implant comprises 40, 41, 42, 43, 44, 45, 46, 47, 48, 49, 50, 51, 52, 53, 54, 55, 56, 57, 58, 59 or 60% by weight of at least one active ingredient as defined above, or a value in the range between any two of the aforementioned values.

The term "anti-inflammatory agent" as used herein, refers to an agent that reduces inflammation.

The term "analgesic", as used herein, refers to any member of the group of drugs used to relieve pain.

The term "steroid" as used herein, refers to an organic compound that contains a specific arrangement of four rings that are joined to each other. Some steroids are also anti-inflammatory agents such as glucocorticoids.

The term "aromatase inhibitor" as used herein, refers to a class of drugs that block the synthesis of estrogens.

The term "gonadotropin-releasing hormone agonist" as used herein, defines a synthetic peptide modeled after the hypothalamic neurohormone gonadotropin-releasing hormone (GnRH) that interacts with the gonadotropin-releasing hormone receptor to elicit its biologic response: the release of the pituitary hormones follicle-stimulating hormone and luteinizing hormone.

In an embodiment, said implant comprises at least one anti-inflammatory agent selected from the group comprising glucocorticoids, non-steroidal anti-inflammatory drugs and immune-selective anti-inflammatory drugs. In an embodiment, said implant comprises at least one glucocorticoid selected from the group comprising hydrocortisone (cortisol), cortisone acetate, prednisone, prednisolone, methylprednisolone, dexamethasone, betamethasone, triamcinolone, beclometasone, fludrocortisone acetate, deoxycorticosterone acetate (DOCA) and aldosterone. In an embodiment, said implant comprises at least one non-steroidal anti-inflammatory drug selected from the group consisting of propionic acid derivatives such as ibuprofen, naproxen, fenoprofen, ketoprofen, flurbiprofen, oxaprozin; acetic acid derivatives such as indomethacin, sulindac, etodolac, ketorolac, diclofenac, nabumetone; enolic acid or oxicam derivatives such as piroxicam, meloxicam, tenoxicam, droxicam, lornoxicam, isoxicam; fenamic acid derivatives such as mefenamic acid, meclofenamic acid, flufenamic acid, tolfenamic acid; and selective COX-2 inhibitors or coxibs such as celecoxib, rofecoxib, valdecoxib, parecoxib, lumiracoxib, etoricoxib and firocoxib. In a preferred embodiment, the implant comprises at least one non-steroidal anti-inflammatory drug selected from celecoxib or sulindac.

In an embodiment, the implant comprises at least one steroid selected from the group comprising estrogens, progestogens, glucocorticoids, androgens and mineralocorticoids; analogs, agonists and antagonists thereof.

In an embodiment, the implant comprises at least one estrogen selected from the group comprising tamoxifen, oestrogen, oestradiol, ethinyl oestradiol, and mestranol.

In an embodiment, the implant comprises at least one progestogen selected from the group comprising progesterone, dienogest, medroxyprogesterone acetate, norgestrel, levonorgestrel, norethindrone, norethindrone acetate, desogestrel, norgestimate, and ethynodiol diacetate. Preferably, said progestogen is dienogest.

In an embodiment, the implant comprises at least one aromatase inhibitor selected from the group comprising atamestane, exemestane, formestane, fadrozole, letrozole, pentrozole, anastrozole, and vorozole.

In an embodiment, the implant comprises at least one gonadotropin-releasing hormone agonist selected from the group comprising leuprorelin, buserelin, gonrelin, triptorelin, nafarelin, deslorelin, histrelin, and supprelin.

In a preferred embodiment, said at least one active ingredient may be selected from the group comprising anastrozole, letrozole, exemestane, dienogest, sulindac and celecoxib. In a more preferred embodiment, said at least one active ingredient may be selected from the group comprising anastrozole, letrozole, exemestane, dienogest, sulindac and celecoxib, and said implant also comprises barium sulfate.

In a preferred embodiment, said implant comprises a PDMS core provided in an EVA tube and a sealant for closure of the open ends of said tube comprising PDMS or a mono-, di-, or triacetoxy derivative thereof, plus at least one active ingredient selected from the group comprising anastrozole, letrozole, exemestane, dienogest, sulindac and celecoxib, and also comprises barium sulfate. In another preferred embodiment, said implant comprises a PDMS core provided in a hydrogel polymer tube and a sealant for closure of the open ends of said tube comprising PDMS or a mono-, di-, or triacetoxy derivative thereof, plus at least one active ingredient selected from the group comprising anastrozole, letrozole, exemestane, dienogest, sulindac and celecoxib, and also comprises barium sulfate. In a further preferred embodiment, said implant comprises a hydrogel polymer core provided in a hydrogel polymer tube and a sealant for closure of the open ends of said tube comprising PDMS or a mono-, di-, or triacetoxy derivative thereof, plus at least one active ingredient selected from the group comprising anastrozole, letrozole, exemestane, dienogest, sulindac and celecoxib, and also comprises barium sulfate. In yet a further preferred embodiment, said implant comprises a PDMS core provided in a hydrogel polymer tube and a sealant for closure of the open ends of said tube comprising a hydrogel polymer, plus at least one active ingredient selected from the group comprising anastrozole, letrozole, exemestane, dienogest, sulindac and celecoxib, and also comprises barium sulfate.

In another aspect, the present invention relates to a method for preparing an implant comprising the steps of:
- preparing a core material comprising PDMS or at least one hydrogel polymer, and at least one active ingredient;
- injecting said core material in a tube comprising an ethylene vinyl acetate polymer or at least one hydrogel polymer;
- curing said core material in said tube;
- closing the open ends of said tube with a sealant comprising PDMS or a mono-, di-, or triacetoxy derivative thereof, or at least one hydrogel polymer, with the proviso that when the sealant is said at least one hydrogel polymer, the core material comprises PDMS.

In a further embodiment, the method for preparing an implant comprises the steps of:
- preparing a mixture of a core material comprising PDMS or at least one monomer precursor of hydrogel, an initiator, a catalyst, a cross-linker and at least one active ingredient;
- injecting said mixture in a tube comprising an ethylene vinyl acetate polymer or at least one hydrogel polymer;
- curing said mixture in said tube;
- closing the open ends of said tube with a sealant comprising PDMS or a mono-, di-, or triacetoxy derivative thereof, or at least one hydrogel polymer, with the proviso that when the sealant is said at least one hydrogel polymer, the core material comprises PDMS.

The term "curing", as used herein, defines the process of hardening a polymer. The catalyst, as used herein, may be selected from the group comprising tin octoate (SnOct₂), platinum-based catalysts and peroxides. In a preferred embodiment, the catalyst is tin octoate (SnOct₂).

In an embodiment, the cross-linker as used herein is an orthosilicate. In a preferred embodiment, the cross-linker is tetrapropyl orthosilicate (SiOPᵣ₄).

Generally, a method for preparing an implant starts with preparing a mixture of a core material comprising PDMS or a (meth)acrylic monomer, a catalyst, a cross-linker and at least one active ingredient selected from an anti-inflammatory agent, a steroid, an aromatase inhibitor or a gonadotropin-releasing hormone agonist. This mixture is then injected into a tube comprising an ethylene vinyl acetate polymer or a hydrogel polymer. After curing of the mixture in the tube, the open ends of the tube are closed with a sealant comprising PDMS or a mono-, di-, or triacetoxy derivative thereof.

In an embodiment, the present invention relates to a method for preparing an implant comprising a PDMS core provided in an EVA tube and a sealant for closure of the open ends of said tube comprising PDMS or a mono-, di-, or triacetoxy derivative thereof. These implants can be synthesized by curing of PDMS in the EVA tube. The method for preparing implants comprising PDMS core in EVA tubes closed with a sealant can start with transferring PDMS and at least one active ingredient into a container. The mixture comprising PDMS and active ingredient can then be placed at temperature below 0°C, for about 5 min to several hours, for example at -20°C for about 1 hour. Then, cross-linker and catalyst can be mixed together in a separate container. The mixture of the catalyst and the cross-linker can then be added into the cold mixture comprising PDMS and active ingredient. The PDMS-active ingredient-cross-linker-catalyst mixture (PDMS mixture) can be homogenized before being preferably placed under vacuum in order to remove the air trapped in the blend. The PDMS mixture can be finally transferred into a dispensing device such as a syringe and kept at temperature below 0°C, for example at -20°C. EVA tubes can be prepared by extrusion of EVA pellets into molds. The PDMS mixture can then be injected into an EVA tube. In an embodiment, the ends of the tube can be closed with a parafilm. After about 12h to 24h of curing at room temperature for example, the tubes can be cut in order to obtain implants of suitable size. The implant extremities can then be closed with PDMS or a mono-, di-, or triacetoxy derivative thereof (also referred herein as adhesive silicone). In an embodiment, the implants can then be subjected to vacuum and/or heat to remove the propanol formed during the PDMS cross-linking.

In a further embodiment, the present invention provides a method for preparing an implant comprising a PDMS core provided in a hydrogel of poly(HEMA) tube and a sealant for closure of the open ends of said tube comprising PDMS or a mono-, di-, or triacetoxy derivative thereof. The method for preparing implants comprising a PDMS core in hydrogel polymer tubes closed with a sealant can start with transferring PDMS and at least one active ingredient into a container. The mixture comprising PDMS and active ingredient can then be placed at temperature below 0°C, for 5 min to several hours, for example mixture can be placed at -20°C for 1 hour. Then, cross-linker and catalyst can be mixed together in a separate container. The mixture of the catalyst and the cross-linker can then be added into the cold PDMS-active ingredient mixture. The PDMS-active ingredient-cross-linker-catalyst mixture (PDMS mixture) can be homogenized before being preferably placed under vacuum in order to remove the air trapped in the blend. The PDMS mixture can be finally transferred into a dispensing device such as a syringe and if necessary kept at temperature below 0°C, for example at -20°C. The tubes of poly(HEMA) can be synthesized by the polymerization of hydroxyethyl methacrylate (HEMA) in a hollow cylinder mold. After repeated wash steps to remove unreacted HEMA, the tubes can be completely dehydrated. This dehydration step can help in completely hardening the tubes and make them resistant to the injection of the PDMS, and also avoids deactivation of the cross-linker, which is sensitive to water. The PDMS can then be injected into the hydrogel tubes. After about 12h to 24h of curing at room temperature for example, the tubes can be cut in order to obtain implants of suitable size. The implant extremities can then be closed with PDMS or a mono-, di-, or triacetoxy derivative thereof. In an embodiment, the implants can then be subjected to vacuum and/or heat to remove the propanol formed during the PDMS cross-linking.

In an embodiment, the present invention relates to a method for preparing an implant comprising at least one hydrogel polymer core provided in a hydrogel polymer tube and a sealant for closure of the open ends of said tube comprising PDMS or a mono-, di-, or triacetoxy derivative thereof. The method for preparing implants comprising a core in hydrogel polymer closed with a sealant can start with transferring to recipient freshly distilled hydroxyethyl methacrylate (HEMA) and at least one active ingredient. In an embodiment, said HEMA can contain 0.1% in weight of ethylene glycol dimethacrylate (EGDMA). The solution can be degassed using an inert gas (such as nitrogen bubbling) and subsequently, ammonium persulfate (APS) aqueous solution and tetramethylethylenediamine (TEMED) can be added. After short homogenization, the solution can be transferred to hollow tubing wherein polymerization occurs. The implants can then be collected. The ends of the implants can then be cut and the implant extremities can then be closed with adhesive silicone. The polyHEMA implants can then be washed by repeated immersion in sterile water to remove unreacted HEMA.

In another embodiment, the method for preparing hydrogel polymer implants closed with a sealant can start with transferring to recipient freshly distilled hydroxyethyl methacrylate (HEMA) and at least one active ingredient into a container. In an embodiment, said HEMA can contain 0.1% in weight of ethylene glycol dimethacrylate (EGDMA). The solution can be degassed using an inert gas (such as nitrogen bubbling) and subsequently, a mixture of potassium persulfate and potassium bisulfite can be added. After short homogenization, the solution can be transferred to hollow tubing wherein polymerization occurs. The implants can then be collected. The ends of the implants can then be cut and the implant extremities can then be closed with adhesive silicone. The polyHEMA implants can then be washed by repeated immersion in sterile water to remove unreacted HEMA.

In a further embodiment, the present invention provides a method for preparing an implant comprising a PDMS core provided in a hydrogel of poly(HEMA) tube and a sealant for closure of the open ends of said tube comprising a hydrogel polymer. The method for preparing implants comprising PDMS core in hydrogel polymer tubes closed with a hydrogel polymer sealant can start with transferring PDMS and at least one active ingredient into a container. The mixture comprising PDMS and active ingredient can be placed at temperature below 0°C, for 5 min to several hours, for example PDMS can be place at -20°C for 1 hour. Then, cross-linker and catalyst can be mixed together in a separate container. The mixture of the catalyst and the cross-linker can then be added into the cold PDMS mixture. The PDMS-cross-linker-catalyst mixture can be homogenized before being preferably placed under vacuum in order to remove the air trapped in the blend. The PDMS mixture can be finally transferred into a dispensing device such as a syringe and if necessary kept at temperature below 0°C, for example at -20°C. The tubes of poly(HEMA) can be synthesized by the polymerization of hydroxyethyl methacrylate (HEMA) in a hollow cylinder mold. After repeated wash steps to remove unreacted HEMA, the tubes can be completely dehydrated. This dehydration step can help in completely hardening the tubes and make them resistant to the injection of the PDMS, and also avoids deactivation of the cross-linker, which is sensitive to water. The PDMS can then be injected into the hydrogel tubes. After about 12h to 24h of curing at room temperature for example, the tubes can be cut in order to obtain implants of suitable size. The implant extremities can then be closed with a hydrogel polymer sealant. The sealant is prepared by transferring to a recipient freshly distilled hydroxyethyl methacrylate (HEMA) and subsequently, adding a mixture of potassium persulfate and potassium bisulfite. After short homogenization, the solution can be transferred to a dispensing device wherein polymerization is allowed to start to increase the viscosity of the solution. The hydrogel sealant can then be used to close the implant extremities. In an embodiment, the implants can then be subjected to vacuum and/or heat to remove the propanol formed during the PDMS cross-linking.

The present invention further relates to a method for preparing an implant as described above comprising the additional step of adding a radiopaque material and/or inert metal coating to the implant. In a particular embodiment, the invention relates to a method for preparing an implant comprising the step of adding at least 0.01% by weight of a radiopaque material to the implant. The invention present invention also encompasses a method for preparing an implant comprising the step of adding at least 0.01 % by weight of a radiopaque material to the core material and/or to the sealant of the implant. The present invention also encompasses a method for preparing an implant comprising the step of coating an implant with at least 0.01% by weight of an inert metal coating.

In a second aspect, the invention provides an implant for use as a medicament. Particularly, the invention provides an implant for use in the treatment of endometriosis.

The present invention provides an implant for use as a medicament, wherein said implant can be administered intraperitoneally or subcutaneously. In a particular embodiment, the present invention provides an implant for use in the treatment of endometriosis, wherein said implant can be administered intraperitoneally or subcutaneously. The subcutaneous administration of the implant is in such a way to ensure the sustained delivery of a therapeutically effective amount of the at least one enclosed active ingredient. The intraperitoneal administration of the implant is in such a way to ensure the localized and sustained delivery of a therapeutically effective amount of the at least one enclosed active ingredient. The term "therapeutically effective amount" as used herein refers to an amount of active ingredient or pharmaceutical agent that elicits the biological or medicinal response in a tissue, system, animal or human that is being sought by a researcher, veterinarian, medical doctor or other clinician, which includes alleviation of the symptoms of the disease being treated.

In an embodiment, the present invention relates to a method for the treatment of endometriosis comprising the step of administering at least one implant according to the invention to an individual in need thereof. In another aspect, the present invention provides a method for the treatment of endometriosis, comprising the step of administering intraperitoneally or subcutaneously at least one implant according to the invention to an individual in need thereof. The term "individual" as used herein refers to a mammal. The individual will preferably be a human.

In a further embodiment, the present invention relates to a method for the treatment of endometriosis, wherein said at least one implant according to the present invention is administered once per 180 days, or less frequently, preferably once per year, or less frequently. The implants of the present invention may be administered at any suitable time interval, preferably once per six months, once yearly, once every 18 months or at any time interval in between, or even less frequently, e.g. every 2-5 years, or even for once only dosing. Typically, the implant is for administration once every 6 months or less frequently. Yet more preferably the composition is for once yearly administration or less frequently. Alternatively, the composition is for once only dosing. The implant may on the other hand be readministered at a later time, in the event of a relapse as defined by symptoms and/or clinical assay.

In an embodiment, the present invention relates to an implantable system comprising: at least one first implant according to the present invention, wherein said at least one first implant comprises at least one active ingredient selected from an anti-inflammatory agent, a steroid, an aromatase inhibitor or a gonadotropin-releasing hormone agonist; and at least one second implant comprising at least one active ingredient selected from an anti-inflammatory agent, a steroid, an aromatase inhibitor or a gonadotropin-releasing hormone agonist. In another embodiment, the present invention relates to an implantable system, as described above, wherein said second implant is also an implant according to the present invention.

In a particular embodiment, the invention provides an implantable system comprising: at least one first implant comprising at least one active ingredient used as an analgesic, selected from an anti-inflammatory agent; and at least one second implant comprising at least one active ingredient able to influence hormone activity, selected from an anti-inflammatory agent, a steroid, an aromatase inhibitor or a gonadotropin-releasing hormone agonist. In an embodiment, the implantable system according to the present invention is designed to include sufficient active agent so as to provide the individual with a required daily dose of a therapeutically effective amount of the active ingredient over the functional useful life of the implants. In a further embodiment, the rate at which the at least one active ingredient is provided from the implantable system to the individual is relatively constant and in such a way to ensure the sustained delivery of a therapeutically effective amount of the at least one enclosed active ingredient.

The present invention also relates to an implantable system, wherein said at least one first implant and said at least one second implant are of a cooperative size and shape and are designed such that each releases a pharmaceutically complementary amount of at least one active ingredient, so as to provide treatment to an individual diagnosed with endometriosis.

The invention will now be illustrated by means of the following synthetic and biological examples, which do not limit the scope of the invention in any way.

### EXAMPLES

### Example 1: Production of polydimethylsiloxane implants

Typically, 19.4 g of polydimethylsiloxane (PDMS, base, medical grade) was placed in a sterile container and kept at -20°C for 1 hour. Thereafter, 0.5 g of tetrapropyl orthosilicate (SiOPᵣ₄, cross-linker, medical grade) and 0.1 g of tin octoate (SnOct₂, catalyst, medical grade) were mixed together in a separate glass container. This mixture of catalyst and cross-linker was then added to the cold PDMS under a laminar flow hood. The PDMS blend was manually mixed for 2 minutes before being placed under a vacuum for 5 minutes in order to remove trapped air bubbles. The PDMS mixture was finally transferred to a plastic syringe and maintained at -20°C.

PDMS implants were prepared by cross-linking the PDMS mixture in a mold at 80°C. This mold, composed of an iron core covered with Teflon film, allows preparation of 12 implants of 20 mm in length and 3 mm in diameter in a row. The PDMS mixture contained in the syringe was injected into the mold, which was then compressed at 80°C under a pressure of 30 bars. After 15 minutes, the mold was cooled at room temperature and the collected implants were transferred to a sterile device. The implants were then placed in a Vismara vacuum oven (VO65) at 950 mbar for 4 hours at room temperature to remove the propanol resulting from the PDMS cross-linking.

### Example 2: Production of ethylene vinyl acetate implants

Ethylene vinyl acetate (EVA) implants were prepared by extrusion of EVA pellets (Elvax 3129, Dupont) in a micro-extruder (DSM 5 cm³ micro-extruder equipped with a twin-screw). For this purpose, EVA pellets were immersed in ethanol in order to extract butyl hydroxytoluene (BHT). After filtration, they were dried under a vacuum at room temperature. Thereafter, 8 g of EVA was introduced into the twin-screw micro-extruder at 80°C at a rotation rate of 100 rpm. After 5 minutes of mixing, the resulting EVA rods were collected and directly placed into sterile water, primarily to fix their geometry, but also to avoid adsorption of dirt onto the surface. The rods were then cut into 2 cm implants and stored in a sterile bag.

### Example 3: Production of poly(hydroxyethyl methacrylate) implants

Typically, 5 ml of freshly distilled hydroxyethyl methacrylate (HEMA) containing 0.1% in weight of ethylene glycol dimethacrylate (EGDMA) was transferred to a glass tube. After degassing the solution by nitrogen bubbling for 5 minutes, 1.67 ml of ammonium persulfate (APS) aqueous solution (APS concentration = 0.024 mol/l) and 7 µl of tetramethylethylenediamine (TEMED) were added. After short homogenization, the solution was transferred to an insulin syringe used as a sterile and disposable mold. The syringes were placed under a laminar flow hood at room temperature for 12 hours to allow polymerization. The implants were then collected by applying simple pressure to the syringe piston. The ends of the implants were cut to obtain implants of 2 cm long (diameter 3 mm). The poly(HEMA) implants were then washed by repeated immersion in sterile water to remove unreacted HEMA. After washing 5 times, the implants were placed in a sterile aqueous solution.

### Example 4: Biocompatibility test of PDMS, EVA and poly(HEMA) implants

Implants were prepared as in example 1, 2 and 3 and the biocompatibility of the 3 polymers, PDMS, EVA and poly(HEMA), was tested in the peritoneal cavity of rats, rabbits and rhesus monkeys. Implants of 20 mm in length and 3 mm in diameter were placed in the peritoneal cavity of 30 rabbits, 30 rats and 3 rhesus monkeys. Inflammation was evaluated by regular hematological analyses and measurement of inflammatory markers such as C-reactive protein and fibrinogen throughout the experiment and by post-mortem examination of the peritoneal cavity. After 3 or 6 months, the animals were euthanized. The implants were macroscopically examined for signs of encapsulation and removed for histological analysis.

Hematological analyses, measurement of inflammatory markers and peritoneal macroscopic examination showed no evidence of inflammation. Histological analysis revealed fibrous tissue encapsulating PDMS and EVA implants in all 3 species and poly(HEMA) implants in rabbits and monkeys. In rats, poly(HEMA) implant surfaces remained relatively free. Calcium deposits were observed inside poly(HEMA) implants in rats and monkeys, but not in rabbits. The results demonstrate that PDMS, EVA and poly(HEMA) polymers are biocompatible in the peritoneal cavity of rats, rabbits and rhesus monkeys.

### Example 5: Synthesis of implants comprising anastrozole as active ingredient according to an embodiment of the invention

The implants comprising a PDMS core were synthesized by curing of PDMS in an ethylene vinyl acetate (EVA) tube. Typically, 19.4 g of PDMS (base, medical grade) and 19.5 g of freshly ground anastrozole (APIN Chemicals Limited, Abingdon, United Kingdom) was transferred into a sterile container before homogenizing the mixture with an ultraturrax T 25 basic (Ika, Staufen, Germany). The blend was placed at -20°C for 1 hour. Then, 0.5 g of tetrapropyl orthosilicate (SiOPᵣ₄, cross-linker, medical grade) and 0.1 g of tin octoate (SnOct₂, catalyst, medical grade) were mixed together in a separate glass container. The mixture of the catalyst and the cross-linker was then added into the cold PDMS mixture inside a laminar flow hood. The PDMS-anastrozole-cross-linker-catalyst mixture was manually homogenized for 2 minutes before being placed under vacuum during 5 minutes in order to remove the air bubbles trapped in the blend. The PDMS mixture was finally transferred into a plastic syringe and kept at -20°C for at least 1 hour.

Typically, EVA tubes were prepared by extrusion of EVA pellets (Elvax 3129, Dupont) in combination with blow molding. The PDMS mixture was then injected into an EVA tube comprising 10% by weight of vinyl acetate (internal diameter of 3 mm, thickness of the wall 200 µm and 15 cm long) in a laminar flow hood. The ends of the tube were closed with a parafilm. After one night of curing at room temperature, the tubes were cut in order to obtain implants of 2 cm long. The implant extremities were closed with MED-2000 adhesive silicone (Nusil technology, Carpinteria, CA, USA). The implants were then moved in a Vismara 65 vacuum oven at 950 mbar for 4 hours at room temperature with the purpose to remove the propanol formed during the PDMS cross-linking.

### Example 6: Synthesis of implants comprising celecoxib as active ingredient according to an embodiment of the invention

The implants comprising a PDMS core were synthesized by curing of PDMS in an ethylene vinyl acetate (EVA) tube. Typically, 19.4 g of PDMS (base, medical grade) and 19.5 g of celecoxib (Kemprotec Limited, Middlesbrough, United Kingdom) was transferred into a sterile container before homogenizing the mixture manually with a stainless spatula. The blend was placed at -20°C for 1 hour. Then, 0.5 g of tetrapropyl orthosilicate (SiOPᵣ₄, cross-linker, medical grade) and 0.1 g of tin octoate (SnOct₂, catalyst, medical grade) were mixed together in a separate glass container. The mixture of the catalyst and the cross-linker was then added into the cold PDMS mixture inside a laminar flow hood. The PDMS-celecoxib-cross-linker-catalyst mixture was manually homogenized for 2 minutes before being placed under vacuum during 5 minutes in order to remove the air bubbles trapped in the blend. The PDMS mixture was finally transferred into a plastic syringe and kept at -20°C for at least 1 hour.

Typically, ethylene vinyl acetate (EVA) tubes were prepared by extrusion of EVA pellets (Elvax 3182, Dupont) in combination with blow molding. The PDMS mixture was then injected into an EVA tube comprising 28 % by weight of vinyl acetate (internal diameter of 3 mm, thickness of the wall 200 µm and 15 cm long) in a laminar flow hood. The ends of the tube were closed with a parafilm. After one night of curing at room temperature, the tubes were cut in order to obtain implants of 2 cm long. The implant extremities were closed with MED-2000 adhesive silicone (Nusil technology, Carpinteria, CA, USA). The implants were then moved in a Vismara 65 vacuum oven at 950 mbar for 4 hours at room temperature with the purpose to remove the propanol formed during the PDMS cross-linking.

### Example 7: Synthesis of implants comprising dienogest as active ingredient according to an embodiment of the invention

Typically, 19.4 g of PDMS (base, medical grade) and 19.5 g of dienogest was transferred into a sterile container before homogenizing the mixture manually with a stainless spatula. The blend was placed at -20°C for 1 hour. Then, 0.5 g of tetrapropyl orthosilicate (SiOPᵣ₄, cross-linker, medical grade) and 0.1 g of tin octoate (SnOct₂, catalyst, medical grade) were mixed together in a separate glass container. The mixture of the catalyst and the cross-linker was then added into the cold PDMS mixture inside a laminar flow hood. The PDMS-dienogest-cross-linker-catalyst mixture was manually homogenized for 2 minutes before being placed under vacuum during 5 minutes in order to remove the air bubbles trapped in the blend. The PDMS mixture was finally transferred into a plastic syringe and kept at -20°C for at least 1 hour.

The PDMS-dienogest mixture was then injected into EVA tubes comprising 10, 18 or 28% by weight of vinyl acetate. The ends of the tube were closed with a parafilm. After one night of curing at room temperature, the tubes were cut in order to obtain implants of 2 cm long. The implant extremities were closed with MED-2000 adhesive silicone (Nusil technology, Carpinteria, CA, USA). The implants were then moved in a Vismara 65 vacuum oven at 950 mbar for 4 hours at room temperature with the purpose to remove the propanol formed during the PDMS cross-linking.

### Example 8: Synthesis of implants comprising sulindac as active ingredient according to an embodiment of the invention

Typically, 19.4 g of PDMS (base, medical grade) and 19.5 g of sulindac (Aldrich) was transferred into a sterile container before homogenizing the mixture manually with a stainless spatula. The blend was placed at -20°C for 1 hour. Then, 0.5 g of tetrapropyl orthosilicate (SiOPᵣ₄, cross-linker, medical grade) and 0.1 g of tin octoate (SnOct₂, catalyst, medical grade) were mixed together in a separate glass container. The mixture of the catalyst and the cross-linker was then added into the cold PDMS mixture inside a laminar flow hood. The PDMS-sulindac-cross-linker-catalyst mixture was manually homogenized for 2 minutes before being placed under vacuum during 5 minutes in order to remove the air bubbles trapped in the blend. The PDMS mixture was finally transferred into a plastic syringe and kept at -20°C for at least 1 hour.

Typically, hydrogel tubes of poly(HEMA) were synthesized by the polymerization of hydroxyethyl methacrylate (HEMA) in a hollow cylinder mold. Typically, 5 ml of freshly distilled hydroxyethyl methacrylate (HEMA) containing 0.1% in weight of ethylene glycol dimethacrylate (EGDMA) was transferred to a glass tube. After degassing the solution by nitrogen bubbling for 5 minutes, 1.67 ml of ammonium persulfate (APS) aqueous solution (APS concentration = 0.024 mol/l) and 7 µl of tetramethylethylenediamine (TEMED) were added. After short homogenization, the solution was transferred into a glass tube (diameter 5 mm). A glass rod (diameter 3 mm) was then placed at the middle of the glass cylinder. After complete polymerization, the poly(HEMA) tube was removed out of the mold. After repeated wash steps to remove the unreacted HEMA, the tubes were completely dehydrated in order to completely harden the tubes and make them resistant to the injection of the PDMS-sulindac mixture, but also to avoid deactivation of the cross-linker, which is sensitive to water. The PDMS-sulindac mixture was then injected into the hydrogel tubes. After one night of curing at room temperature, the tubes were cut in order to obtain implants of 2 cm long.

### Example 9: Synthesis of implants comprising barium sulfate in the core material

Typically, a mixture of 19.4 g of PDMS (base, medical grade) and 1 g, 2 g or 4 g of barium sulfate (5, 10 and 20% by weight, Aldrich) was transferred into a sterile container in a laminar flow hood. The mixture was homogenized with a spatula and placed at -20°C for 1 hour. Tubes comprising EVA (10% by weight of vinyl acetate, thickness of the wall 200 µm) were washed with water, dried and sterilized by UV in a laminar flow hood. Then, 0.5 g of SiOPᵣ₄ and 0.1 g of SnOct₂ were mixed together in a separate glass container. The mixture of the catalyst and the cross-linker was then added into the cold PDMS-barium sulfate mixture inside a laminar flow hood. After mixing the catalyst-cross-linker and the PDMS-barium sulfate, the mixture was injected in the EVA tubes. The ends of the tube were closed with a parafilm and left for one night in the laminar flow hood. After polymerization, the tubes were cut in order to obtain implants of 2 cm long. The implant extremities were closed with MED-2000 adhesive silicone (Nusil technology, Carpinteria, CA, USA).

### Example 10: Synthesis of implants comprising anastrozole as active ingredient and barium sulfate in the core material according to an embodiment of the invention

Typically, a mixture of 19.4 g of PDMS (base, medical grade), 19.5 g of freshly ground anastrozole (APIN chemicals Limited, Abingdon, United Kingdom) and 2 g, 4 g or 8 g of BaSO₄ (5, 10 and 20% by weight of barium sulfate, Aldrich) was transferred into a sterile container in a laminar flow hood. The mixture was homogenized with a spatula and placed at -20°C for 1 hour. Tubes comprising EVA (10% by weight of vinyl acetate, internal diameter of 3 mm, thickness of the wall 200 µm and 15 cm long) were washed with water, dried and sterilized by UV in a laminar flow hood. After mixing the catalyst-cross-linker and the PDMS-anastrozole-barium sulfate as described above, the mixture was injected in the EVA tubes. After polymerization, the tubes were cut in order to obtain implants of 2 cm long. The implant extremities were closed with MED-2000 adhesive silicone (Nusil technology, Carpinteria, CA, USA).

### Example 11: Synthesis of implants comprising anastrozole as active ingredient and barium sulfate in the sealant according to an embodiment of the invention

Typically, 19.4 g of PDMS (base, medical grade) and 19.5 g of freshly ground anastrozole (APIN Chemicals Limited, Abingdon, United Kingdom) was transferred into a sterile container before homogenizing the mixture with an ultraturrax T 25 basic (Ika, Staufen, Germany). The blend was placed at -20°C for 1 hour. Then, 0.5 g of tetrapropyl orthosilicate (SiOPᵣ₄, cross-linker, medical grade) and 0.1 g of tin octoate (SnOct₂, catalyst, medical grade) were mixed together in a separate glass container. The mixture of the catalyst and the cross-linker was then added into the cold PDMS mixture inside a laminar flow hood. The PDMS-anastrozole-cross-linker-catalyst mixture was manually homogenized for 2 minutes before being placed under vacuum during 5 minutes in order to remove the air bubbles trapped in the blend. The PDMS mixture was finally transferred into a plastic syringe and kept at -20°C for at least 1 hour.

Typically, ethylene vinyl acetate (EVA) tubes were prepared by extrusion of EVA pellets (Elvax 3129, Dupont) into molds. The PDMS mixture was then injected into an EVA tube comprising 10% by weight of vinyl acetate (internal diameter of 3 mm, thickness of the wall 200 µm and 15 cm long) in a laminar flow hood. The ends of the tube were closed with a parafilm. After one night of curing at room temperature, the tubes were cut in order to obtain implants of 2 cm long. MED-2000 adhesive silicone (Nusil technology, Carpinteria, CA, USA) was mixed with increasing amounts of BaSO₄ (20% and 50% by weight). The implant extremities were closed with the MED-2000 mixtures. The implants were then moved in a Vismara 65 vacuum oven at 950 mbar for 4 hours at room temperature with the purpose to remove the propanol formed during the PDMS cross-linking.

### Example 12: Radiopacity of the implants

### Implants comprising barium sulfate in the core material:

PDMS implants comprising barium sulfate in the core material (5, 10 and 20% by weight) were prepared as in example 9. Typically, 19.4 g of PDMS (base, medical grade) and 1 g, 2 g or 4 g of BaSO₄ (Aldrich) were transferred into a sterile container before homogenizing the mixture with a stainless spatula. The blend was placed at -20°C for 1 hour. Then, 0.5 g of tetrapropyl orthosilicate (SiOPᵣ₄, cross-linker, medical grade) and 0.1 g of tin octoate (SnOct₂, catalyst, medical grade) were mixed together in a separate glass container. The mixture of the catalyst and the cross-linker was then added into the cold PDMS mixture inside a laminar flow hood. The PDMS-anastrozole-cross-linker-catalyst mixture was manually homogenized for 2 minutes before being placed under vacuum during 5 minutes in order to remove the air bubbles trapped in the blend. The PDMS mixture was finally transferred into a plastic syringe and kept at -20°C for at least 1 hour.

Typically, ethylene vinyl acetate (EVA) tubes were prepared by extrusion of EVA pellets (Elvax 3129, Dupont) in combination with blow molding. The PDMS mixture was then injected into an EVA tube comprising 10% by weight of vinyl acetate (internal diameter of 3 mm, thickness of the wall 200 µm and 15 cm long) in a laminar flow hood. The ends of the tube were closed with a parafilm. After one night of curing at room temperature, the tubes were cut in order to obtain implants of 2 cm long. The implant extremities were closed with MED-2000 adhesive silicone (Nusil technology, Carpinteria, CA, USA). The implants were then moved in a Vismara 65 vacuum oven at 950 mbar for 4 hours at room temperature with the purpose to remove the propanol formed during the PDMS cross-linking.

### Implants comprising barium sulfate in the sealant:

PDMS implants comprising barium sulfate in the sealant (20 and 50% by weight) were prepared as in example 11, but without the active ingredient. Typically, 19.4 g of polydimethylsiloxane (PDMS, base, medical grade) was placed in a sterile container and kept at -20°C for 1 hour. Then, 0.5 g of tetrapropyl orthosilicate (SiOPᵣ₄, cross-linker, medical grade) and 0.1 g of tin octoate (SnOct₂, catalyst, medical grade) were mixed together in a separate glass container. The mixture of the catalyst and the cross-linker was then added into the cold PDMS inside a laminar flow hood. The PDMS-cross-linker-catalyst mixture was manually homogenized for 2 minutes before being placed under vacuum during 5 minutes in order to remove the air bubbles trapped in the blend. The PDMS mixture was finally transferred into a plastic syringe and kept at -20°C for at least 1 hour.

Typically, ethylene vinyl acetate (EVA) tubes were prepared by extrusion of EVA pellets (Elvax 3129, Dupont) in combination with blow molding. The PDMS mixture was then injected into an EVA tube comprising 10% by weight of vinyl acetate (internal diameter of 3 mm, thickness of the wall 200 µm and 15 cm long) in a laminar flow hood. The ends of the tube were closed with a parafilm. After one night of curing at room temperature, the tubes were cut in order to obtain implants of 2 cm long. MED-2000 adhesive silicone (Nusil technology, Carpinteria, CA, USA) was mixed with increasing amounts of BaSO₄ (20 and 50% by weight). The implant extremities were closed with the MED-2000 mixtures. The implants were then moved in a Vismara 65 vacuum oven at 950 mbar for 4 hours at room temperature with the purpose to remove the propanol formed during the PDMS cross-linking.

### Radiopacity:

Five types of implants were synthesized comprising 5, 10 or 20% of barium sulfate in the core material or comprising 20 or 50% of barium sulfate in the sealant and were X-rayed. Figure 1 represents an X-ray image of an implant 1 comprising 20% of barium sulfate in the sealant, of an implant 2 comprising 50% of barium sulfate in the sealant, of an implant 3 comprising 5% of barium sulfate in the core material and of an implant 4 comprising 10% of barium sulfate in the core material. Figure 2 represents an X-ray image of in vitro implant 5 comprising 20% of barium sulfate in the sealant and of an implant 6 comprising 20% of barium sulfate in the core material.

The radiopacity of the implants was tested in vivo in cynomolgus monkeys. Abdominal incisions were made and 2 implants were inserted without fixation. One implant was placed at the right side and one implant was placed at the left side, after which the skin was sutured. Figures 3 represent X-ray images of an implant 7 comprising 50% of barium sulfate in the sealant, placed at the right side and of an implant 8 comprising 20% of barium sulfate in the sealant, placed at the left side. Figure 3A represents a front view of the animal on day 0. Figure 3B represents a side view of the animal on day 0. Figures 4 represent X-ray images of an implant 9 comprising 20% of barium sulfate in the core material, placed at the right side and of an implant 10 comprising 50% of barium sulfate in the sealant, placed at the left side. Figure 4A represents a front view of the animal on day 0. Figure 4B represents a side view of the animal on day 0. Figure 4C represents a front view of the animal after 2 months. Figure 4D represents a side view of the animal after 2 months. Figures 5 represent X-ray images of 2 implants without barium sulfate, an implant 11 comprising 5% of barium sulfate in the core material, an implant 12 comprising 10% of barium sulfate in the core material and of an implant 13 comprising 20% of barium sulfate in the core material. Figure 5A represents a front view of the animal on day 0. Figure 5B represents a side view of the animal on day 0. The implants comprising barium sulfate in the core material or sealant were visible. The implants without barium sulfate were not detected.

### Example 13: Effect on the release of anastrozole by using MED-2000 adhesive silicone as a sealant

Implants comprising anastrozole as an active ingredient were prepared as described in example 5, but with and without MED-2000 adhesive silicone as a sealant. Implants were tested for the release of anastrozole without a sealant and with MED-2000 adhesive silicone as a sealant. Furthermore, the implants comprised an EVA tube comprising 10% by weight of vinyl acetate and a thickness of 0.1 mm, 0.2 mm or 0.3 mm. In order to determine the kinetics of the release of anastrozole, the implants were placed in sealed tubes comprising 100 ml of phosphate buffer pH 7.4. The tubes were placed in a bath at 37°C and 140 rpm. The measurements were performed during 400 days for the implants without a sealant and during more than 500 days for the implants with MED-2000 adhesive silicone as a sealant. Figure 6A represents the mean release of anastrozole per 24h in function of the time for implants without a sealant. The results show that during the first 5 days an important quantity of anastrozole is released from the implants followed by a decreasing quantity of released anastrozole per 24h in function of time. Figure 6B represents the mean release of anastrozole per 24h in function of the time for implants with MED-2000 adhesive silicone as a sealant. The results show that the release of anastrozole from the implants is constant during time for more than 400 days.

### Example 14: Effect of sterilization on the release of anastrozole

Implants were prepared as in example 5. Two series of 3 implants were sterilized with ethylene oxide in order to see if sterilization influenced the release of the active principle. Sterilization was performed before placing the implants in the sealed tubes comprising 100 ml of phosphate buffer pH 7.4. The tubes were placed in a bath at 37°C and 140 rpm. The measurements were performed during 14 days. Figure 7 illustrates the mean release of anastrozole per 24h in function of the time for sterilized and non-sterilized implants with MED-2000 adhesive silicone as a sealant. The results show that the sterilization has no significant effect on the release of anastrozole.

### Example 15: Release of celecoxib from implants with or without a sealant:

Three series of 3 implants were prepared, the first series was implant without sealant, the second was implant with EVA as a sealant and the last one was implants with PDMS as a sealant in order to see if the sealant influenced the release of the active ingredient. The implants were placed in the sealed tubes comprising 100 ml of phosphate buffer pH 7.4. The tubes were placed in a bath at 37°C and 140 rpm. The measurements were performed during 38 days. Figure 8A illustrates the mean release of celecoxib per 24h in function of the time for implant without sealant. Figure 8B illustrates the mean release of celecoxib per 24h in function of the time for implant with EVA as a sealant. Figure 8C illustrates the mean release of celecoxib per 24h in function of the time for implant with PDMS as a sealant.

The results show that the sealant has significant effect on the release of Celecoxib and that PDMS sealant can reduce the burst effect and controlled the liberation of the celecoxib.

### Example 16: Effect of the composition of the tube on the release of celecoxib

Implants were prepared comprising PDMS as a core material and celecoxib as an active ingredient without a tube or with an EVA tube comprising 18% or 28% by weight of vinyl acetate and with MED-2000 adhesive silicone as a sealant. In order to determine the kinetics of the release of celecoxib, the implants were placed in sealed tubes comprising 100 ml of phosphate buffer pH 7.4. The tubes were placed in a bath at 37°C and 140 rpm. The measurements were performed during 400 days for all implants tested. Figures 9A and 9B represent the mean release of celecoxib per 24h in function of the time for implants without a tube and with an EVA tube comprising 18% or 28% by weight of vinyl acetate. The results show that the release of celecoxib is constant for more than 300 days for implants with an EVA tube comprising 28% by weight of vinyl acetate. The release of celecoxib is also constant for more than 300 days for implants with an EVA tube comprising 18% by weight of vinyl acetate. The results show that the implants comprising 18% by weight of vinyl acetate release a lower amount of celecoxib per day compared with implants with an EVA tube comprising 28% by weight of vinyl acetate. The implants without an EVA tube show an exponential decrease in the release of celecoxib.

### Example 17: Pharmacokinetic study of implants comprising a PDMS core and celecoxib as active ingredient in Wistar rats

Implants comprising celecoxib as active ingredient were synthesized as described in example 6. 28 of these implants were placed intraperitoneal in Wistar rats and 28 were placed subcutaneous in Wistar rats. One implant was used per rat. Pharmacokinetics was observed for more than 6 months. Six rats were used to determine the concentration of celecoxib in the serum. The concentration of celecoxib in the serum was determined every day during the first week, 2 times per week during the next 3 weeks and once per week during the following weeks of the study. At every time point, two rats were sampled. 16 rats were used to measure the concentration of celecoxib in the peritoneal cavity. The peritoneal concentration of celecoxib was assayed on day 1, day 4 and once per month during the next months of the study. At every time point, 2 rats were sampled and sacrificed. The peritoneal concentration of celecoxib was determined in a 1 ml sample obtained after washing the peritoneal cavity with 1 ml of phosphate buffer pH 7.4. The results shown in Figure 10 demonstrate that the active ingredient celecoxib is liberated in a controlled way during more than 6 months. Figure 10A illustrates the concentration of celecoxib in serum in function of the number of days after implantation. No differences in the concentration of celecoxib in the serum were observed between rats receiving the implant intraperitoneal and rats receiving the implant subcutaneous. Figure 10B illustrates the concentration of celecoxib in peritoneal liquid in function of the number of days after implantation. The results show that the concentration of celecoxib in the peritoneal liquid is higher when the implant is delivered intraperitoneally compared with subcutaneous implantation.

### Example 18: Pharmacokinetic study of implants comprising a PDMS core and anastrozole as active ingredient in Wistar rats

Implants comprising anastrozole as active ingredient were synthesized as described in example 5. 28 of these implants were placed intraperitoneal in Wistar rats and 28 were placed subcutaneous in Wistar rats. One implant was used per rat. Pharmacokinetics was observed for more than 6 months. Six rats were used to determine the concentration of anastrozole in the serum. The concentration of anastrozole in the serum was determined every day during the first week, 2 times per week during the next 3 weeks and once per week during the following weeks of the study. At every time point, 2 rats were sampled. 16 rats were used to measure the concentration of anastrozole in the peritoneal cavity. The peritoneal concentration of anastrozole was assayed on day 1, day 4 and once per month during the next months of the study. At every time point, 2 rats were sampled and sacrificed. The peritoneal concentration of anastrozole was determined in a 1 ml sample obtained after washing the peritoneal cavity with 1 ml of phosphate buffer pH 7.4. Figure 11A illustrates the concentration of anastrozole in serum in function of the number of days after intraperitoneal implantation. The results demonstrate that the concentration of anastrozole is constant for more than 6 months in the serum of rats after intraperitoneal implantation. Figure 11B illustrates the concentration of anastrozole in peritoneal fluid in function of the number of days after intraperitoneal implantation. The results demonstrate that the concentration of anastrozole is constant for 6 months in the peritoneal fluid of rats after intraperitoneal implantation.

### Example 19: Pharmacokinetic study of implants comprising a PDMS core and celecoxib or anastrozole as active ingredient in cynomolgus monkeys

Implants comprising anastrozole or celecoxib as active ingredient were prepared as described in examples 5 and 6. Two cynomolgus monkeys received each two implants comprising celecoxib and 2 cynomolgus monkeys received each two implants comprising anastrozole. Pharmacokinetics was observed for more than 5 months for celecoxib and for more than 10 months for anastrozole. The concentration of the active ingredient in the serum was determined every 3 days during the first week, once per week during the next 3 months and once per month during the remainder of the study. Figure 12A illustrates the concentration of celecoxib in the serum in function of the number of days after implantation. Figure 12B illustrates the concentration of anastrozole in serum in function of the number of days after implantation. The results show that the implants comprising anastrozole or celecoxib effectively liberate their active ingredient in a controlled way during the desired period.

### Example 20: Analysis of the remaining amounts of anastrozole in the implants in vivo

Implants comprising anastrozole as active ingredient were prepared as described in examples 5. The implants were placed subcutaneous or intraperitoneal and the remaining amounts of anastrozole in the implants were determined at different time points after implantation. Figure 13 demonstrates the remaining amounts of anastrozole in the implants placed subcutaneous or intraperitoneal. The result shows that the amount of anastrozole in the implants placed subcutaneous or intraperitoneal after 180 days was still satisfying; suggesting that a long term delivery of the active ingredient is possible.

## Claims

1. An implant comprising:
- a core material comprising polydimethylsiloxane or at least one hydrogel polymer;
- a tube encasing said core material comprising an ethylene vinyl acetate polymer or at least one hydrogel polymer;
- a sealant for closure of the open ends of said tube comprising polydimethylsiloxane or a mono-, di-, or triacetoxy derivative thereof, or at least one hydrogel polymer; and
- at least one active ingredient;
with the proviso that when the sealant is said at least one hydrogel polymer, the core material comprises polydimethylsiloxane.

2. The implant according to claim 1, wherein said implant comprises an inert metal coating and/or at least one radiopaque material, preferably said implant comprises at least 0.01% by weight of an inert metal coating and/or at least 0.01% of at least one radiopaque material.

3. The implant according to claim 2, wherein said radiopaque material is selected from the group comprising barium, gold, platinum, tantalum, bismuth and iodine or salts thereof, or a radiopaque polymer, preferably said radiopaque material is barium sulfate.

4. The implant according to claim 2, wherein said inert metal is selected from the group comprising silver, gold, titanium, tungsten, barium, bismuth, platinum and palladium.

5. The implant according to any of claims 1 to 4, wherein said at least one active ingredient is selected from an anti-inflammatory agent, a steroid, an aromatase inhibitor or a gonadotropin-releasing hormone agonist.

6. The implant according to claim 5, wherein said anti-inflammatory agent is selected from celecoxib and sulindac.

7. The implant according to claim 5, wherein said steroid is selected from the group comprising estrogens, progestogens, glucocorticoids, androgens and mineralocorticoids; analogs, agonists and antagonists thereof.

8. The implant according to claim 7, wherein said estrogen is selected from the group comprising tamoxifen, oestrogen, oestradiol, ethinyl oestradiol, and mestranol.

9. The implant according to claim 7, wherein said progestogen is selected from the group comprising dienogest, progesterone, medroxyprogesterone acetate, norgestrel, levonorgestrel, norethindrone, norethindrone acetate, desogestrel, norgestimate, and ethynodiol diacetate.

10. The implant according to claim 5, wherein said gonadotropin-releasing hormone agonist is selected from the group comprising leuprorelin, buserelin, gonrelin, triptorelin, nafarelin, deslorelin, histrelin, and supprelin.

11. The implant according to any of claims 1 to 10, wherein said implant comprises from about 40% to about 75% by weight of said at least one active ingredient.

12. The implant according to any of claims 1 to 11, for use as a medicament.

13. The implant according to any of claims 1 to 12, for use in the treatment of endometriosis.

14. The implant according to any of claims 12 or 13, wherein said implant is administered intraperitoneally or subcutaneously.

15. The implant according to any of claims 12 to 14, wherein said implant is administered once per 180 days, or less frequently, preferably once per year, or less frequently.
